# EUROPEAN PATENT APPLICATION

(11) **EP 1 857 053 A2**
(43) Date of publication of application: **21.11.2007**
(21) Application number: 07252085.1
(22) Date of filing: 21.05.2007
(51) Int. Cl.: A61B 17/02

(54) **Lumen stabilizer for endoscopic micosal resection**

(30) Priority: 19.05.2006 US 438131
(71) Applicant: ETHICON ENDO-SURGERY, INC., Cincinnati, Ohio 45242 (US)
(72) Inventor: Lu, Ifung, Skokie, IL 60076 (US); Nobis, Rudolph H., Mason, OH 45040 (US); Linenkugel, Duane A., Cincinnnati, OH 45249 (US)
(74) Representative: Tunstall, Christopher Stephen

(57) **Abstract**

An endoscopic instrument having a lumen stabilizing feature, including a flexible shaft having a first distal end for insertion into the gastrointestinal tract of a patient, a plurality of arm housings disposed about the periphery of the distal end, a plurality of arms retained within the arm housings, wherein the arms may be extended from and retracted into the arm housings; and a finger connected to an arm, wherein the finger may be rotated with respect to a lateral axis of the arm. Lumen stabilizing accessories for flexible endoscopic instruments are also disclosed.

## Description

### FIELD OF THE INVENTION

The present application relates to endoscopic instruments and accessories and, more particularly, to stabilization aids such as manipulators, sheaths and stents for the stabilization of internal tissues in endoscopic surgical procedures.

### BACKGROUND OF THE INVENTION

Various endoscopic procedures have been developed to treat early stage cancers of the gastrointestinal tract, including protruded and superficial lesions developing within the tissues of the esophagus, stomach and colon. Such cancers may present as pre-cancerous or cancerous lesions of the mucosal layer and may include margins that abut or partially penetrate the submucosal layer of such tissues. In such circumstances, a surgeon may wish to remove the pre-cancerous or cancerous lesion by performing a microsurgical operation generally categorized as an endoscopic mucosal resection ("EMR"). Such an operation typically requires the surgeon to introduce a flexible endoscope, such as a colonoscope or a gastroscope, through a natural orifice in communication with the patient's gastrointestinal tract and guide the distal end of the instrument to the site of a lesion using the visualization capabilities of the instrument. The surgeon may then resect the lesion and a margin of healthy tissue, including portions of the surrounding mucosa and underlying submucosa, through one of a number of microsurgical procedures.

For example, such resections may be performed using a "lift-and-cut" procedure that employs a pair of forceps and a cauterizing wire snare extended through separate working channels of a dual channel endoscope. The lift-and-cut procedure generally includes the steps of injecting a solution beneath the submucosa adjacent to a lesion to separate the mucosa and submucosa from the underlying muscularis; placing a wire snare around the lesion on the surface of the mucosa; grasping the lesion through the wire snare with a pair of forceps; and operating the wire snare to excise the lesion. The "lift-and-cut" procedure and variants that employ a wire snare advantageously do not require highly specialized instruments, but disadvantageously tend to be ill-suited for the resection of large lesions, where divided resections may increase the risks of incomplete resection and recurrence of disease.

Such resections may also be performed using a circumferential resection procedure that employs an endoscopic needle knife, hook knife, insulation-tipped diathermic knife (IT knife) or the like and, optionally, a wire snare. The circumferential procedure generally includes the steps of injecting a solution beneath the submucosa adjacent to a lesion to separate the mucosa and submucosa from the underlying muscularis; creating a circumferential incision around the lesion with an endoscopic knife; creating a series of stepwise lateral incisions under the circumscribed tissue between the submucosa and muscularis to separate the circumscribed tissue from the muscularis; and optionally, if the lesion is attached by a pedicle, guiding a wire snare around the circumferentially separated lesion and operating the wire snare to sever the pedicle. The circumferential procedure may advantageously permit the collection of large en bloc specimens for the assessment of complete or incomplete resection, which may reduce the risks of incomplete resection and recurrence of disease, but may disadvantageously include an increased risk of perforation of the muscularis. The circumferential procedure also presently requires a substantial amount of time to complete, e.g., a range of about 1 to about 2 hours per lesion. The reader will appreciate that the risk of perforation of the muscularis, and possibly the time required to complete the procedure, might be reduced if a surgeon were able to establish a greater degree of control over the pliable tissues that comprise the majority of the esophagus and colon.

Such resections may also be performed using specialized devices, such as the improved EMR device disclosed in U.S. Ser. No. filed April 28, 2006 (Docket No. END5794USNP) entitled "Apparatus and Method for Performing an Endoscopic Mucosal Resection," the entire contents of which are incorporated herein by reference. Briefly, such a device may include a pair of pivotable arms bearing cutting elements which are inserted between the submucosa and the muscularis adjacent to a lesion. The device permits a surgeon to bluntly dissect a lesion and underlying submucosal layer away from the muscularis through a scissor-like manipulation of the arms and cutting elements. After the dissection is complete, the surgeon may reorient the cutting elements perpendicularly or partially transversely with respect to the plane of dissection and resect the dissected lesion and submucosa through further manipulation of the cutting elements. This improved device also advantageously permits the collection of large en bloc specimens for the assessment of complete or incomplete resection, which may reduce the risks of incomplete resection and recurrence of disease, but may in some instances require a surgeon to establish extraordinary control over the surgical site and surrounding tissues. The term "extraordinary control" this context describes the application of an apparatus and/or method that places the tissue at the surgical site under localized tension, such that the mucosa, submucosa, and muscularis adjacent to a lesion are presented as a set of substantially smooth and non-convoluted layers.

EMR is generally performed without the use of stabilization aids, and in procedures such as the "lift-and-cut" procedure described earlier, such aids may not provide substantial assistance. However, as more complicated manual procedures are developed and more complicated surgical devices come into use, it may become desirable to establish a greater degree of control over the soft and pliable tissues of the gastrointestinal tract. Such stabilization aids may assist surgeons seeking to perform complicated manual procedures such as circumferential resection, or those seeking to employ semi-automatic resection devices such as the referenced improved EMR device. Such stabilization aids may also permit surgeons performing endoscopic procedures to adapt some of the more complex techniques employed in open surgery for microsurgical use.

Accordingly, there is a need for endoscopic instruments that provide stabilization aids that may be deployed from the distal end of the instrument and recovered in association with the instrument at the conclusion of a procedure. In addition, there is a need to stabilization accessories that may be operated in combination with a general purpose endoscopic instrument providing auxiliary device channels to support varying surgical roles.

### SUMMARY OF THE INVENTION

One aspect of the disclosed lumen stabilizer may include a flexible shaft having a first distal end for insertion into the gastrointestinal tract of a patient, a plurality of arm housings disposed about the periphery of the distal end, a plurality of arms retained within the arm housings, wherein the arms may be extended from and retracted into the arm housings; and a finger connected to an arm, wherein the finger may be rotated with respect to a lateral axis of the arm.

Another aspect of the disclosed lumen stabilizer may include a control handle, an arm actuator connected to the control handle ,a finger actuator connected to the control handle and coaxially retained within the arm actuator, an arm connected to the arm actuator, and a finger connected to the arm and the finger actuator, wherein the finger may be rotated with respect to a lateral axis of the arm.

In yet another aspect, the disclosed lumen stabilizer may include a mounting sheath, a flexible stent stabilizer having a proximal portion of the stent stabilizer affixed to a distal portion of the mounting sheath and a constraining sheath slideably interconnected with and overlapping the mounting sheath, wherein the constraining sheath may be slid over and past the distal portion of the mounting sheath to compress the flexible stent stabilizer.

Other aspects of the disclosed lumen stabilizer will become apparent from the following description, the accompanying drawings and the appended claims.

### BRIEF DESCRIPTION OF THE DRAWINGS

Fig. 1 is a perspective view of one aspect of the disclosed lumen stabilizer device;

Fig. 2(a) is a perspective view of a portion of the lumen stabilizer device shown in Fig. 1, in a first configuration;

Fig. 2(b) is a perspective view of a portion of the lumen stabilizer device shown in Fig. 1, in a second configuration;

Fig. 2(c) is a perspective view, partially in section, of the portion of the lumen stabilizer device shown in Fig. 2(a);

Fig. 3 is a perspective side of a second aspect of the disclosed lumen stabilizer device;

Fig. 4(a) is a perspective view of a portion of the lumen stabilizer device shown in Fig. 3, in a first configuration;

Fig. 4(b) is a perspective view of a portion of the lumen stabilizer device shown in Fig. 3, in a second configuration;

Fig. 4(c) is a perspective view, partially in section, of the portion of the lumen stabilizer device shown in Fig. 4(a);

Fig. 5 is a side elevational view, in section, of a third aspect of the disclosed lumen stabilizer device; and

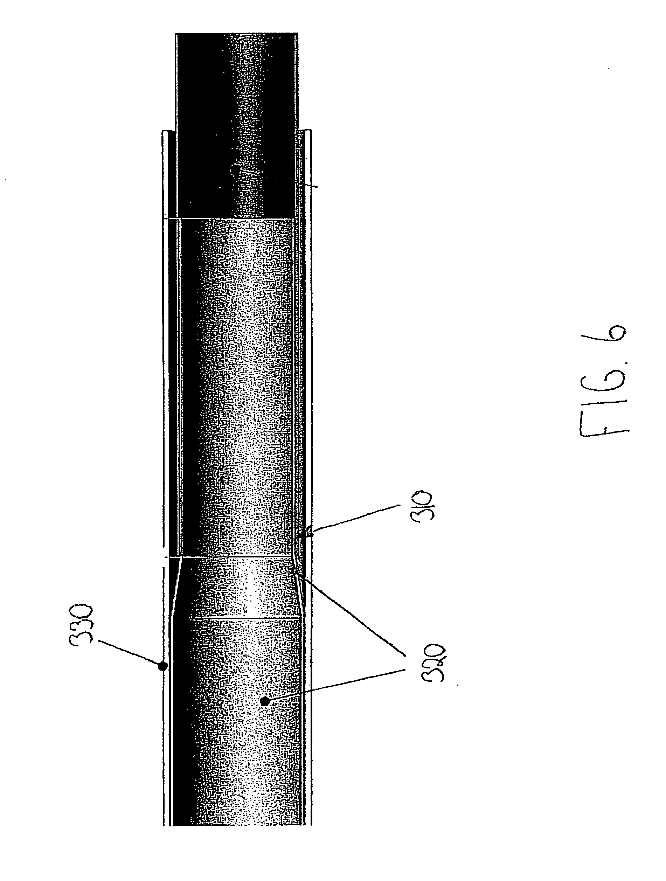

Fig. 6 is a side elevational view, in section, of a portion of the lumen stabilizer accessory shown in Fig. 5.

### DETAILED DESCRIPTION OF THE INVENTION

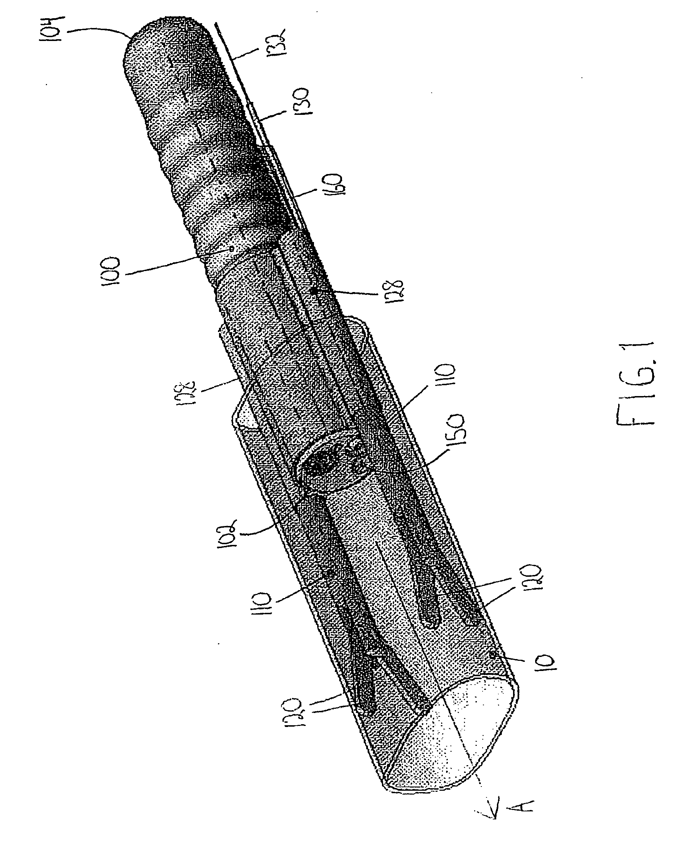

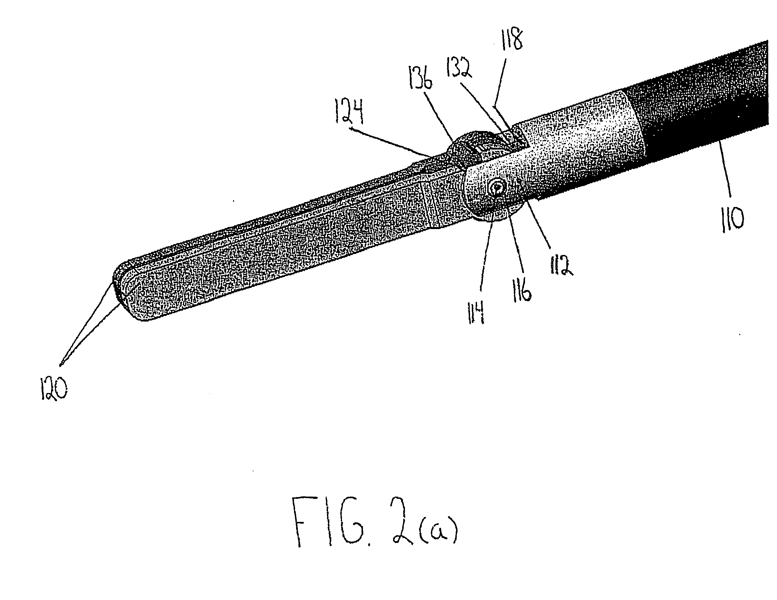

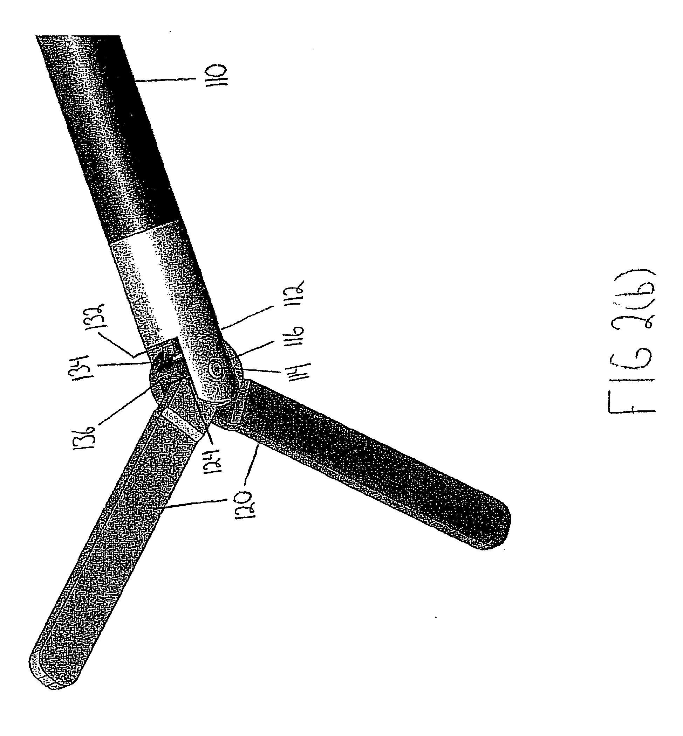

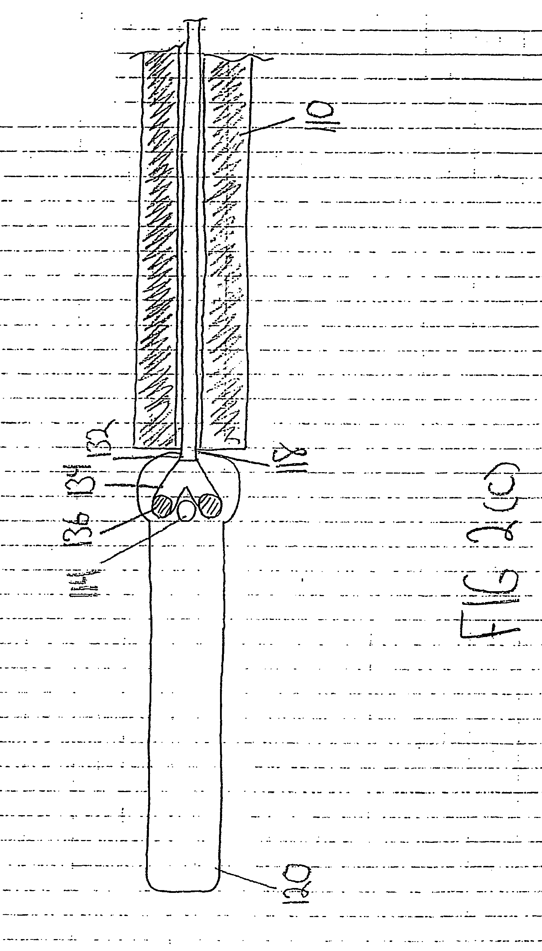

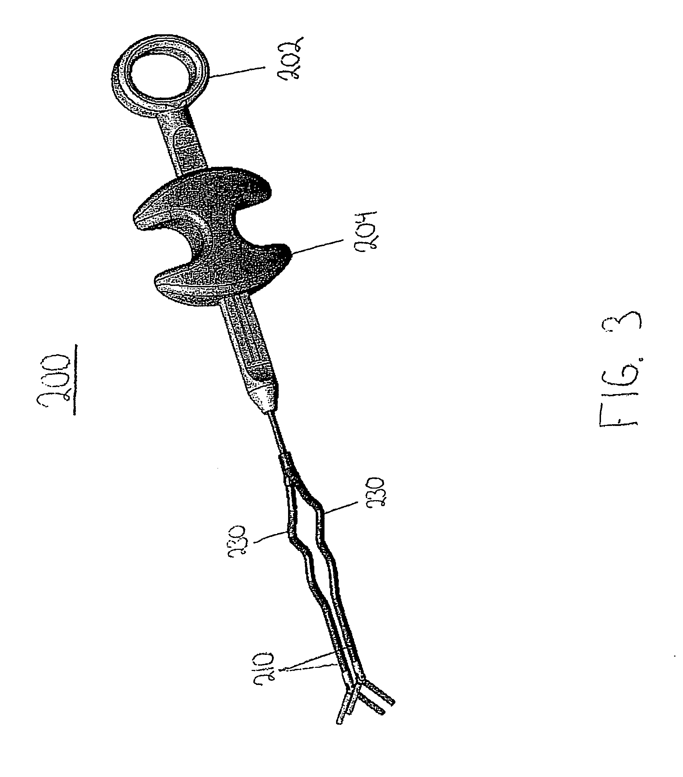

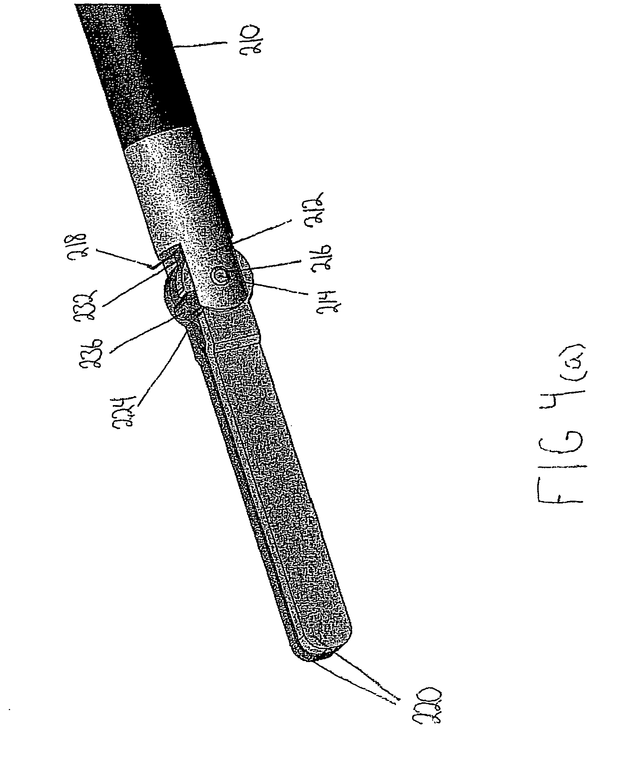

Referring to Fig. 1, a first aspect of an endoscopic instrument is shown and may include a flexible shaft 100 having a distal end 102 for insertion into the gastrointestinal tract 10 of a patient and a proximal end 104 for the connection of light sources, fiberscopic eyepieces, camera displays, irrigation lines, vacuum sources, actuator controls and the like. The shaft 100 may be sized and shaped to received a flexible endoscope therein and may define an elongated axis A, which may be linear or curvilinear. The distal end 102 of flexible shaft 100 may include a plurality of arms 110 that may be extended from and retracted into a plurality of arm housings 128 disposed about the periphery of distal end 102.

The flexible shaft 100 may also include a working channel 150 and a plurality of auxiliary channels 160. The channels 150, 160 may be either internal or external of the shaft 100. Working channel 150 may be used during surgical procedures to deploy endoscopic devices such as needles, forceps, snares, knives, and other such devices known in the art. An auxiliary channel 160 may house an arm actuator 130 that provides a means for extending and retracting an arm 110 in relation to protective sleeve 128. Arm actuator 130 may be connected to arm 110 by gluing, welding, or other methods known in the art. Arm actuator 130 may also coaxially house a finger actuator 132 (e.g., an actuation cable). In such case, arm actuator 130 may be provided as a helically wound stainless steel wire sheath, and finger actuator 132 may be provided as a stainless steel wire slidingly movable within that sheath. Arm actuator 130 and finger actuator 132 may be independently manipulated by various trigger controls, plunger controls, slider controls or other control means attached to proximal end 104 of flexible shaft 100.

With reference to Figs. 2(a), 2(b) and 2(c), each arm 110 may include a finger actuator channel 118 and a distal fork 112, which may further include a pivot pin 114 and a pair of pivot pin receivers 116. Finger actuator channel 118 may house a portion of finger actuator 132 extending distally from arm actuator 130. Finger actuator 132 may terminate in a Y-shaped head 134 providing offset and diverging actuator pins 136. When finger actuator 132 is fully extended distally from finger actuator channel 118, head 134 may abut pivot pin 114, and when finger actuator 132 is fully retracted proximally into finger actuator channel 118, head 134 may abut arm 110. A pivoting finger 120 may be provided which may include a hub 122 that encircles pivot pin 114 and an offset actuator pin receiver 124 that receives and interlocks with an actuator pin 136 on head 134. A pair of fingers 120 may be anchored within distal fork 112 on either side of head 134 by inserting pivot pin 114 between pivot pin receivers 116 and through hub 122. The actuator pin receivers 124 and actuator pins 136 may drive fingers 120 to rotate around pivot pin 114, such that when finger actuator 132 is extended distally, fingers 120 may close together and become aligned with arm 110 (Fig. 2(a)), and when finger actuator 132 is retracted proximally, fingers 120 may spread apart with respect to the arm 110 (Fig. 2(B)).

Accordingly, arms 110 and fingers 120 may be operated to apply localized tension to the tissues of a tubular structure such as the esophagus or colon. Arm actuators 130 may be extended distally to deploy arms 110 from arm housings 128 arrayed around distal end 102 of a flexible endoscope, which may part some of the tissue adjacent to distal end 102 of the instrument, and finger actuators 132 may be retracted proximally to spread apart fingers 120 to the deployed configuration, creating a perimeter that may approximate the distended perimeter of a tubular tissue such as an esophagus or colon. Such distension may place the tissues under a moderate degree of tension, such that the mucosa, submucosa and muscularis adjacent to arms 110 and distal end 102 are presented as a set of substantially smooth and non-convoluted layers. When a surgeon desires to reposition or remove the flexible endoscope, finger actuators 132 may be extended distally to close fingers 120, and arm actuators 130 may be retracted proximally to withdraw arms 110 within arm housings 128, releasing the tissues and readying the instrument for movement within the patient.

Referring to Fig. 3, a first aspect of an endoscopic accessory is shown for use with a flexible endoscopic instrument having a plurality of peripheral auxiliary channels (not shown), such as a flexible endoscope. Such endoscopic devices may include conventional flexible endoscopes combined with "overtubes" that add peripheral auxiliary channels to instruments that otherwise lack them. The accessory 200 may include a plurality of arms 210 that may be extended from and retracted into the distal openings of the auxiliary channels of the instrument. Each arm 210 may be connected to a control handle 202 by way of an arm actuator 230 that provides a means for extending and retracting arms 210 in relation to the auxiliary channels of the endoscope. Arm actuator 230 may be connected to arm 210 by gluing, welding, or other methods known in the art. Arm actuator 230 may also coaxially house a finger actuator 232. In such case, arm actuator 230 may be provided as a helically wound stainless steel wire sheath, and finger actuator 232 may be provided as a stainless steel wire slidingly movable within that sheath. The actuators 230 and 232 may be routed through the aforementioned auxiliary channels and connected to a control handle 202 disposed outside the proximal auxiliary channel openings in the instrument. A surgeon or surgical assistant may move control handle 202 towards the proximal auxiliary channel openings to extend arm actuators 230 from the distal auxiliary channel openings of the instrument, and away from the proximal auxiliary channel openings to retract arm actuators 230 into the distal auxiliary channel openings of the instrument.

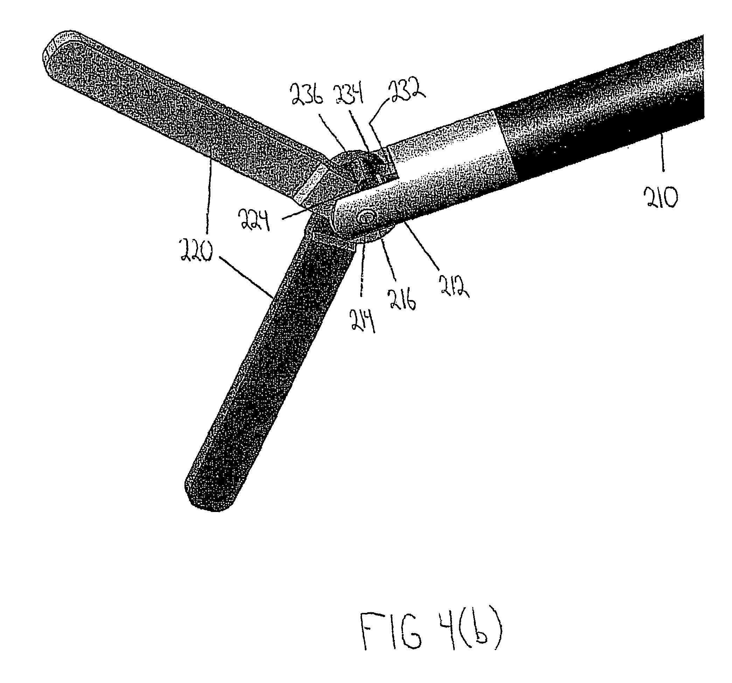

With reference to Figs. 4(a), 4(b) and 4(c), each arm 210 may include a finger actuator channel 218 extending therethrough and a distal fork 212. The distal fork 212 may include a pivot pin 214 and a pair of pivot pin receivers 216. Finger actuator channel 218 may house a portion of finger actuator 232 extending distally from arm actuator 230. Finger actuator 232 may terminate in a Y-shaped head 234 providing offset and diverging actuator pins 236. When finger actuator 232 is fully extended distally from finger actuator channel 218, head 234 may abut pivot pin 214, and when finger actuator 232 is fully retracted proximally into finger actuator channel 218, head 234 may abut arm 210. A pivoting finger 220 may be provided which may include a hub 222 that encircles pivot pin 214 and an offset actuator pin receiver 224 that receives and substantially encloses an actuator pin 236 on head 234. A pair of fingers 220 may be anchored within distal fork 212 on either side of head 234 by inserting pivot pin 214 between pivot pin receivers 216 and through hub 222. The actuator pin receivers 224 and actuator pins 236 drive fingers 220 to rotate around pivot pin 214, such that when finger actuator 232 is extended distally, fingers 220 may close together and become aligned with arm 210, and when finger actuator 232 is retracted proximally, fingers 120 may spread apart with respect to arm 210. The surgeon or surgical assistant may manipulate a slider control 204 in control handle 202 (Fig. 3) to operate fingers 220, sliding the control 204 towards the distal end of the control handle 202 to distally extend finger actuators 232 and close fingers 220, aligning fingers 220 with arms 210, and sliding control 204 towards the proximal end of the control handle 202 to proximally retract finger actuators 232, spreading fingers 220 with respect to arms 110.

Accordingly, arms 210 and fingers 220 may be operated to apply localized tension to the tissues of a tubular structure such as the esophagus or colon. Control handle 202 may be manipulated to distally extend arms 210 from the distal auxiliary channel openings in a flexible endoscopic instrument, which may part some of the tissue adjacent to the distal end of the instrument, and sliding control 204 may be manipulated to spread fingers 120. The extension of arms 210 and spreading of fingers 220 may create a perimeter that may approximate the distended perimeter of a tubular tissue such as an esophagus or colon. Such distension may place the tissues under a moderate degree of tension, such that the mucosa, submucosa and muscularis adjacent to arms the distal end of the flexible endoscopic instrument are presented as a set of substantially smooth and non-convoluted layers. When a surgeon desires to reposition or remove the flexible endoscope, sliding control 204 may be manipulated to close fingers 220, aligning fingers 220 with arms 210, and control handle 202 may be manipulated to retract arms 210 within the distal auxiliary channel openings of the flexible endoscopic instrument.

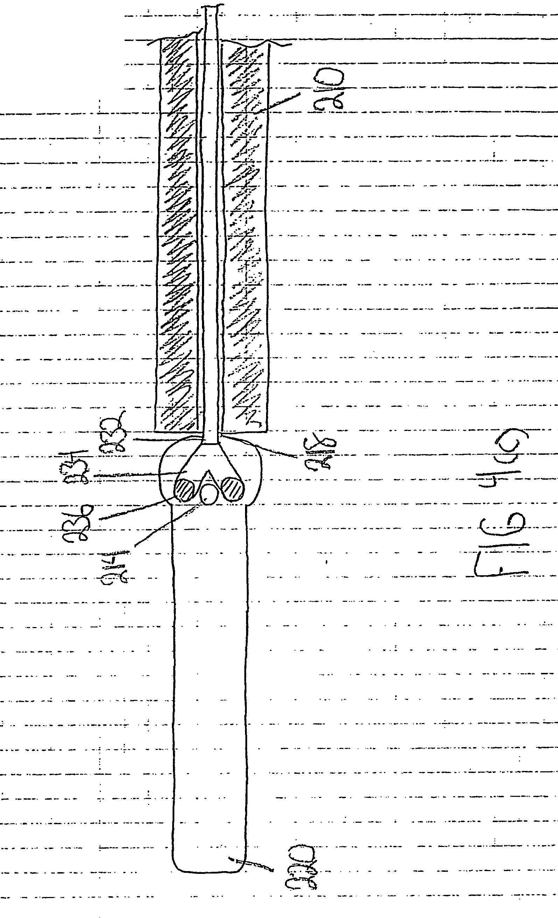

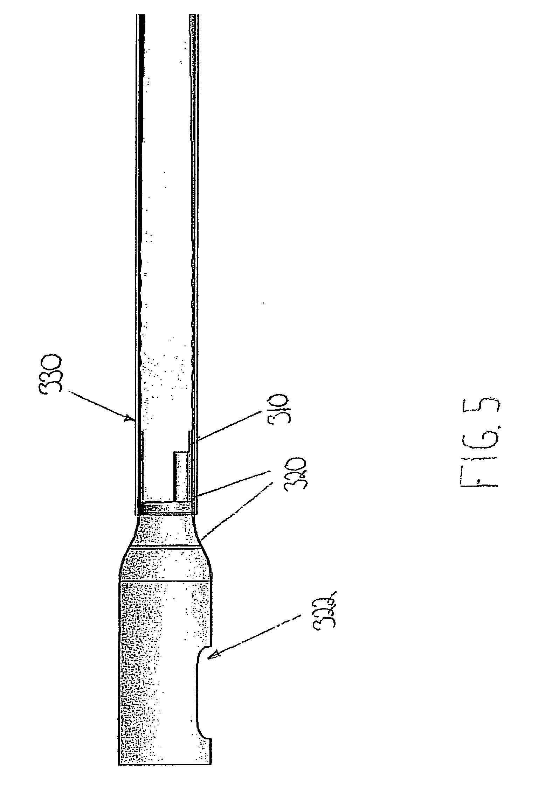

With reference to Fig. 5, another aspect of a lumen stabilizer adapted for use with a flexible endoscopic instrument, such as a gastroscope or a colonoscope, is shown. The device 300 may include a mounting sheath 310, a stent stabilizer 320 and constraining sheath 330. Mounting sheath 310 may be configured as a hollow tube having a generally annular cross section adapted to interlock with the distal end of a flexible endoscopic instrument. Such an interlocking relationship may be established by configuring the inner surface of mounting sheath 310 to conform to the ridged outer surface of a flexible endoscope, by affixing a portion of mounting sheath 310 to the outer surface of a flexible endoscope using a clamp, or by other means that will be apparent to those having skill in the art. A proximal end of stent stabilizer 320 may be affixed to the distal end of mounting sheath 310, such that a substantial portion of stent stabilizer 320 extends beyond the distal end of mounting sheath 310. Stent stabilizer may be affixed to mounting sheath 310 by welding, gluing, clamping or other means known in the art. Constraining sheath 330 may be configured as a hollow tube adapted to conform to the shape of mounting sheath 310 and to be slidably operable with respect to mounting sheath 310. The distal end of constraining sheath 330 may be further adapted to define a gap between the inner wall of constraining sheath 330 and the outer wall of mounting sheath 310, the gap providing an annular volume that may receive the proximal end of stent stabilizer 320.

Stent stabilizer 320 may be both expandable and collapsible and may be adapted or otherwise pre-stressed such that the stent may assume an expanded configuration when it is neither compressed nor constrained by adjacent structures. Stent stabilizer may be designed to have an expanded diameter that is greater than the diameter of the distal end of the flexible endoscopic instrument, and to have an collapsed diameter that is approximately equal to the diameter of the mounting sheath 310. The stent stabilizer 320 may be manufactured from a cylindrical mesh of resilient material such as nylon or, optionally, from a highly resilient material exhibiting a shape-memory effect, such as nickel-titanium alloy (nitinol). The mesh of stent stabilizer 320 may optionally be modified to provide an operative window 322 within the weave of the mesh, which may provide devices operated from the distal end of an endoscopic instrument with access to tissues abutting the outer surface of the stent.

Constraining tube 330 may be slidably operated with respect to the mounting sheath 310 to collapse or release stent stabilizer 320. If constraining tube 330 is configured as an overtube extending proximally along the shaft of a flexible endoscopic instrument, constraining tube 330 may be manipulated by a control mounted at the proximal end of the instrument. Alternately, if constraining tube 330 is configured as a comparatively short sheath, constraining tube 330 may be connected to an actuator cable extending alongside the shaft of the flexible endoscopic instrument. Constraining tube 330 may be advanced distally past the distal end of mounting sheath 310, whereby interference between constraining tube 330 and stent stabilizer 320 will tend to cause stent stabilizer 320 to collapse to a diameter that is approximately equal to the diameter of mounting sheath 310. Constraining tube 330 may also be retracted proximally along mounting sheath 310 and the distal end of the flexible endoscopic instrument, whereby the distal end of stabilizing stent 320 may be released and may expand to a diameter that is substantially greater than the diameter of the distal end of the flexible endoscopic instrument. The expansion of a distal portion of stabilizing stent 320 may create a perimeter barrier that may approximate the distended perimeter of a tubular tissue such as an esophagus or colon. Such distension may place the tissues under a moderate degree of tension, such that the mucosa, submucosa and muscularis adjacent to the distal end of the stabilizing stent 320 and, optionally, within operative window 322, are presented as a set of substantially smooth and non-convoluted layers.

Although various aspects of the disclosed device have been shown and described herein, modifications may occur to those skilled in the art upon reading this specification. The present application includes such modifications as are within the spirit of the invention, and is to be limited only by the scope of the appended claims.

## Claims

1. An endoscopic device for stabilizing a lumen comprising:
an elongated flexible shaft defining an elongated axis, said shaft including a distal end and a proximal end;
at least one arm having a distal end and a proximal end, said proximal end of said arm being connected to said distal end of said shaft;
a first finger pivotally connected to said distal end of said arm; and
a second finger pivotally connected to said distal end of said arm and moveable relative to said first finger from a first configuration, wherein said first and second fingers are generally aligned with said axis, to a second configuration, wherein said first and second fingers extend at an angle relative to said axis.

2. The device of claim 1 wherein said shaft if sized to receive an endoscope therein.

3. The device of claim 1 wherein said arm is moveably connected to said distal end of said shaft.

4. The device of claim 1 further comprising an arm housing connected to said distal end of said shaft, wherein said arm is retractable within said arm housing.

5. The device of claim 1 wherein said arm is generally aligned with said axis.

6. The device of claim 1 further comprising a finger actuator operatively connected to at least one of said first and second arms.

7. The device of claim 6 wherein said finger actuator is an actuation cable.

8. The device of claim 6 wherein said finger actuator extends through said proximal end of said shaft.

9. The device of claim 6 wherein actuation of said finger actuator urges said first and second fingers from said first configuration to said second configuration.

10. A lumen stabilizing device for an endoscopic instrument having auxiliary channels comprising:
a control handle;
an arm actuator connected to said control handle;
a finger actuator connected to said control handle and coaxially retained within said arm actuator;
an arm connected to said arm actuator, said arm defining an arm axis; and
a finger pivotally connected to said arm and said finger actuator and pivotably from a first configuration, wherein said finger is generally aligned with said arm axis, to a second configuration, wherein said finger is disposed at an angle relative to said arm axis.

11. A lumen stabilizing device for an endoscopic instrument having auxiliary channels, the device comprising:
a mounting sheath having a proximal end and a distal end;
a flexible stent stabilizer connected to said distal end of said mounting sheath; and
a constraining sheath slideably received over said mounting sheath,
wherein said constraining sheath may be urged in a distal directed to apply a compressing force to said flexible stent stabilizer.

12. The device of claim 11 wherein said flexible stent stabilizer is biased to a deployed, expanded configuration.

13. The device of claim 11 wherein said flexible stent stabilizer is generally cylindrical in shape.

14. The device of claim 11 wherein said flexible stent stabilizer is formed from a mesh material.
